# EUROPEAN PATENT SPECIFICATION

(11) **EP 1 859 276 B1**
(45) Date of publication and mention of the grant of the patent: **14.12.2011**
(21) Application number: 06709889.7
(22) Date of filing: 24.02.2006
(51) Int. Cl.: G01N 33/53, G01N 33/569, G01N 33/573

(54) **DETECTION OF LIPID OXIDISING ABZYMES IN SAMPLES**
NACHWEIS LIPIDOXIDIERENDER ABZYME IN PROBEN
DÉTECTION D'ABZYMES D'OXYDATION DES LIPIDES DANS DES ÉCHANTILLONS

(30) Priority: 25.02.2005 GB 0503940
(43) Date of publication of application: 28.11.2007
(73) Proprietor: Cambridge Theranostics Limited, Cambridge, Cambridgeshire CB2 4AT (GB)
(72) Inventor: PETYAEV, Ivan Cambridge Theranostics Limited, Cambridge Cambridgeshire CB2 4AT (GB)
(74) Representative: Sutcliffe, Nicholas Robert
(86) International application number: PCT/GB2006/000657
(87) International publication number: WO 2006/090164

(56) References cited:
- WO-A-03/017992
- WO-A-03/019198

## Description

This invention relates to the detection of lipid oxidising anti-Chlamydia abzymes in samples of blood or serum. This is useful, for example, in assessing an individual for cardiovascular conditions.

Lipid oxidation is one of the main processes leading to the conversion of circulating lipoproteins into highly atherogenic factors [Goto Y. (1982) supra, Halliwell B., and J.M.C. Gutteridge, (1989) supra, Schultz D., and Harrison D.G. (2000) supra]. The principal cause of lipid oxidation is catalytic antibodies known as 'abzymes' (see WO03/017992, WO03/019196 and WO03/019198). Abzymes are a key pathogenic factor in the development of atherosclerosis and are an important diagnostic marker for atherosclerosis-related conditions as well as being a target for therapeutic intervention.

WO03/019198 proposes screening methods for inhibitors of lipid-oxidising anti-Chlamydia abzymes.

WO03/017992 proposes the treatment of atherosclerotic conditions by inhibiting lipid-oxidising anti-Chlamydia abzymes.

Current abzyme assays are based on the measurement of the oxidation of lipids by the abzymes. The amount of lipid oxidation is, for example, determined from the level of lipid oxidation product malondialdehyde (MDA). MDA levels can be conveniently measured spectrophotometrically, since a coloured product forms when malondialdehyde reacts with thiobarbituric acid [Draper, H.H. et al Free Radic. Biol. Med. (1993) 15, 353].

However, abzyme assays based on lipid oxidation are slow, because the development of lipid oxidation products generally requires 8-12 hours. Lipid oxidation assays also require the use of toxic reagents, such as trichloroacetic acid, and large volumes of patient serum (typically 2-3 ml).

The present inventor has recognised that abzymes damage Chlamydia antigens and this damage can be used to measured abzyme activity in simple, rapid assays using conventional immunoassay formats.

An aspect of the invention provides a method of measuring anti-Chlamydia abzyme levels in a sample comprising;
abrogating or abolishing anti-Chlamydia abzyme-mediated lipid oxidation activity in the sample, without affecting the binding of abzymes or anti-Chlamydia antibodies to Chlamydia antigens, and
determining the binding of antibodies in the sample to a Chlamydia antigen.

An increase in binding in the sample relative to untreated controls is indicative of the presence of abzymes in the sample. The amount of the increase is indicative of the level of abzymes in the sample. The absence of any increase in binding in the sample relative to controls is indicative of the absence of abzymes from the sample.

The binding of antibodies in samples treated to abolish abzyme mediated lipid oxidation activity and in controls (i.e. untreated samples) may be determined simultaneously or sequentially.

In some embodiments, an initial sample taken from an individual may be divided or aliquoted into two or more separate samples, at least one of which is treated to abrogate or abolish abzyme mediated lipid oxidation and at least one of which remains untreated as a control. In other embodiments, two or more identical samples may be obtained from the individual, at least one of which is treated to abrogate abzyme mediated lipid oxidation and at least one of which remains untreated as a control.

The presence of anti-Chlamydia abzymes in the serum of an individual may be indicative of the individual having or suffering from an atherosclerotic disorder or may be indicative of the susceptibility or risk of the individual suffering from such a disorder in the future. The amount, level or activity of abzymes may be indicative of the severity or level of risk of the disorder i.e. an increase in the amount and/or activity of antibody is indicative of increased severity or risk of disorder.

A method of assessing an individual for an atherosclerotic disorder may comprise;
abrogating or abolishing anti-Chlamydia abzyme mediated lipid oxidation activity in a sample obtained from the individual without affecting the binding of abzymes or anti-Chlamydia antibodies to Chlamydia antigens; and,
determining the binding of antibodies in the sample to a Chlamydia antigen.

The increase in binding in the treated sample relative to untreated sample is indicative of the level of abzymes in the sample. The presence, severity or susceptibility of the individual to an atherosclerotic disorder may be assessed from the level of abzymes in the sample.

Also described are methods which may be useful, for example, in determining the optimal therapeutic treatment for an individual. For example, an individual having anti-lipid abzymes indicative of an atherosclerotic condition may be subjected to therapeutic treatment to alleviate the condition or its symptoms. The level or amount of anti-lipid abzymes may be indicative of the severity of the condition and may be used to determine whether or not a particular therapeutic course is appropriate.

A sample is preferably a sample which comprises plasma or serum from the individual, for example a blood, serum or plasma sample. Methods for obtaining, storing and preparing suitable samples from an individual are well known in the medical practice. A test sample of serum may be obtained, for example, by extracting blood from an individual and isolating the serum from the extracted blood. Suitable extraction methods include centrifugation to separate serum and plasma from cellular material.

A Chlamydia antigen may be any immunogen or immunogenic component of a Chlamydia cell i.e. a molecule from Chlamydia which evokes or is capable of evoking an immune response in a mammal against the Chlamydia cell, for example a molecule on the surface of a Chlamydia cell, or a mimetic or functional analogue of such a component. In other words, the Chlamydia antigen is a component of a Chlamydia cell that is capable of specifically binding to antibodies raised against the Chlamydia cell. Suitable Chlamydia antigens for use in the present methods include isolated, purified, cloned or synthesised antigens of Chlamydia, fragments or epitopes of such antigens, groups of such antigens, fraction(s) of Chlamydia cell homogenate, whole Chlamydia cells, and combinations of any of these.

Anti-idiotypic antibodies which imitate active Chlamydia epitopes or fragments of these anti-idiotypic antibodies, may also be suitable for use as Chlamydia antigens in the present methods.

Suitable methods for preparing Chlamydia antigens are well known in the art. For example, Chlamydia antigens may be isolated and/or purified by techniques such as HPLC.

In some preferred embodiments, the Chlamydia antigen may be on the surface of a Chlamydia cell and methods described herein may comprise determining the binding of antibodies in treated and untreated sample to the Chlamydial cell.

A Chlamydial cell may be a cell from a species belonging to the Chlamydia psittaci group. The Chlamydia psittaci group includes Chlamydia psittaci and Chlamydia pneumoniae. In some embodiments, the Chlamydial cell may be an ovine Chlamydia psittaci cell. Suitable preparations of live ovine Chlamydia psittaci in a lyophilised form are available commercially (Intervet).

The binding of antibodies in a sample to a Chlamydia antigen may be determined by any appropriate means or assay format. Tagging with individual reporter molecules is one possibility. For example, a second antibody which binds to antibodies in the sample, or a Chlamydia cell or antigen may be tagged with a reporter molecule. The reporter molecules may directly or indirectly generate detectable, preferably measurable, signals. Where required, linkage of reporter molecules may be direct or indirect, covalent, e.g. via a peptide bond, or noncovalent. Linkage via a peptide bond may be as a result of recombinant expression of a gene fusion encoding binding molecule (e.g. antibody) and reporter molecule.

Reporters include fluorochromes such as fluorescein, rhodamine, phycoerythrin and Texas Red, chromogenic dyes such as diaminobenzidine, macromolecular colloidal particles or particulate material such as latex beads that are coloured, magnetic or paramagnetic, and biologically or chemically active agents that can directly or indirectly cause detectable signals to be visually observed, electronically detected or otherwise recorded.

Biologically or chemically active agents include enzymes which catalyse reactions that develop or change colour or cause changes in electrical properties. Agents may be molecularly excitable, such that electronic transitions between energy states result in characteristic spectral absorptions or emissions. They may include chemical entities used in conjunction with biosensors. Biotin/avidin or biotin/streptavidin and alkaline phosphatase detection systems may be employed. Further examples include horseradish peroxidase and chemiluminescence. Any such method may be used to determine the binding of the antibody to Chlamydia antigen.

The signals generated by individual antibody-reporter conjugates may be used to derive quantifiable absolute or relative data of the relevant antibody binding in samples (normal and test).

Methods of the invention may be carried out in any convenient format. Immunological assays are well-known in the art and many suitable formats are available and may be employed to carry out the present methods, for example ELISA, Western blotting, microimmunofluorescence (MIF), Biacore®, (Biacore, Upsala, Sweden), immunoprecipitation or immuno-turbidimetry, agglutination, for example erythrocyte-, latex- or other polymer-based agglutination, immunohistochemistry, immunoelectrophoresis, antibody-based affinity chromatography and IDEIA^{®} (Boots-Celltech) and other red-ox amplifying diagnostic systems.

In some preferred embodiments, a sandwich assay format may be employed. For example, sandwich assay may employ a capture antibody that binds anti-Chlamydia antibodies in the sample and a labelled Chlamydia antigen or cell that detects the presence of anti-Chlamydia antibodies bound to the capture antibody. Alternatively, a sandwich assay may employ a capture Chlamydia antigen or cell and a labelled antibody which detects the presence of anti-Chlamydia antibodies bound to the antigen. A capture antibody, Chlamydia antigen or Chlamydia cell may be immobilised, for example, by attachment to an insoluble support or solid surface. The support may be in particulate or solid form and may include a plate, a test tube, beads, a ball, a filter or a membrane. Methods for fixing antibodies to insoluble supports are known to those skilled in the art. A non-immobilised component of an assay (i.e. a component which is free in solution) such as an antibody, Chlamydia antigen or Chlamydia cell may comprise a detectable label as described above. For example, the antibody may be labeled with a fluorophore such as FITC or rhodamine, a radioisotope, or a non-isotopic labelling reagent such as biotin or digoxigenin; components containing biotin may be detected using "detection reagents" such as avidin conjugated to any desirable label such as a fluorochrome.

The mode of determining binding is not a feature of the present invention and those skilled in the art are able to choose a suitable mode according to their preference and general knowledge.

The sample may be treated to abrogate abzyme mediated lipid oxidation using any convenient physical or a chemical treatment which abolishes or substantially reduces abzyme activity but which has no effect or substantially no effect on the binding of abzymes to Chlamydia antigens.

In some embodiments, the sample may be physically treated to inactivate abzyme mediated lipid oxidation.

For example, the sample may be heated. The sample may be heated in accordance with any temperature regimen that inactivates lipid oxidation activity but does not affect binding properties of specific antibodies.

A suitable temperature regimen may include heating the sample to at least 37°C, or at least 56°C. The sample may be heated for a sufficient time to inactivate or substantially inactivate abzyme mediated lipid oxidation without affecting the binding of abzymes to antigen. For example the sample may be heated for at least 1 minute, at least 5 minutes, at least 15 minutes, at least 45 minutes, at least 60 minutes, at least 8 hours, at least 12 hours, or at least 24 hours.

In some embodiments, the sample may be heated to 56°C for at least 15, at least 20, at least 30, at least 45, or at least 60 minutes; or heated to 37°C for at least 8 hours, at least 12 hours, at least 24 hours, or at least 48 hours.

Other physical treatments may be used to inactivate lipid oxidation activity without affecting the binding properties of specific antibodies.

The sample may be subjected to repetitive freeze-thaw cycles, for example two or more cycles of freezing followed by thawing.

The sample may be subjected to prolonged storage, for example at least 4 days at 0°C to 4°C, at least 2 or at least 3 months at -10° or at least 4 or at least 6 months at - 20°C.

The sample may be subjected to high-energy ultrasound, microwave, UV, gamma radiation or any other electro magnetic waves.

The suitability of a treatment or regimen for use in the present methods may be determined by measuring the lipid oxidation and Chlamydia-binding activity of the sample after treatment, as described herein. A suitable treatment or regimen for use in the present methods inactivates abzyme mediated lipid oxidation but does not affect the binding properties of anti-Chlamydia antibodies;

In other embodiments, the sample may be chemically treated to inactivate abzyme mediated lipid oxidation. For example, the sample may be treated with one or more abzyme inactivating agents.

Inactivating agents may include low pH antioxidants (i.e. inhibits oxidation reactions at pH5.5), hydroxyl radical scavengers, `electron trappers' such as crown ethers and steroids, 'electron cushions' such as polyvinyl-based polymers, `electron sinks', such as ubiquinones and Q₈, copper chelators and calcium chelators.

Suitable inactivating agents may include ascorbic acid, acetyl salicylic acid, sodium azide, catechins, including catechin gallate, DMSO, azithromycin, haemoglobin or telithromycin.

In other embodiments, an inactivating agent may be a bacterial cell, for example a cell from probiotic bacteria such as lactobacilli, or a product of such a cell.

The efficacy of a treatment in inactivating abzymes may be determined by determining lipid oxidation activity of a sample of abzymes, for example IgG obtained from a patient atheroma, before and after treatment. Any convenient method of determining lipid oxidation may be used. Many methods for determining lipid oxidation are known in the art and may be used to determine the reduction or abrogation of lipid oxidation activity in a sample. Suitable methods are, for example, described in CRC Handbook of Methods for Oxygen Radical Research, CRC Press, Boca Raton, Florida (1985), Oxygen Radicals in Biological Systems. Methods in Enzymology, v. 186, Academic Press, London (1990); Oxygen Radicals in Biological Systems. Methods in Enzymology, v. 234, Academic Tokyo (1996) In preferred embodiments, oxidation is determined by determining the production (i.e. the presence or amount) of a lipid oxidation product, which may include aldehydes such as malondialdehyde (MDA), (lipid) peroxides, diene conjugates or hydrocarbon gases.

The sample from the individual may be treated to inhibit or reduce complement activity. In some embodiments, the sample may be treated with a complement inhibitor. Inhibitors of complement activity are well known in the art and include, for example, Ca²⁺ chelators such as EGTA or EDTA, thymidine kinase inhibitors, including catechins such as epigallocatechin gallate (EGCG), polysaccarides such as zymosan, peptidyl molecules such as CD46, CD55, CD59, pexelizumab, eculizumab, compstatin, Cobra venom, antibodies against C1q and other components or intermediates of complement cascade, and fragments of these antibodies, and compounds which imitate the functions and properties of the complement cascade.

In other embodiments, the sample may be treated with a procedure or regimen that inhibits complement activity. Suitable procedures include heating the sample, for example to 56°C for 30 minutes, or other temperature regimen that inactivates complement. Other physical procedures, such as ultrasound shock, irradiation and/or laser treatment, may also be used.

The methods of determining abzyme activity that are described herein may also be useful in screening for compounds which inhibit abzyme activity. Such compounds may be useful in the treatment of atherosclerotic conditions.

Another aspect of the invention provides a method of screening for an anti-Chlamydia abzyme inhibitor comprising;
determining the binding of antibodies in a sample to a Chlamydia antigen,

Another aspect of the invention provides a method of screening for an inhibitor of anti-Chlamydia abzyme-mediated lipid oxidation comprising;
determining the binding of antibodies in a sample to a Chlamydia antigen,
wherein the sample comprises lipid oxidising anti-Chlamydia abzymes,
treating the sample with a test compound, wherein the test compound does not affect the binding of abzymes or antibodies to a Chlamydia antigen, and;
determining the binding of antibodies in the treated sample to a Chlamydia antigen.

An increase in binding to a Chlamydia antigen after said treatment is indicative that the compound is an abzyme inhibitor.

The sample is preferably a sample comprising abzymes. A suitable sample may be obtained from the individual having an atherosclerotic disorder and may, for example, be a serum sample or sample from anatheroma or atherosclerotic lesion. The sample may be enriched for IgG, for example by binding with protein A, as described below. The presence of abzymes in the sample may be confirmed using lipid oxidation assays which are known in the art.

An abzyme inhibitor identified by these methods may be useful in the treatment of a atherosclerotic disorder, including a cardiovascular disorder such as atherosclerosis, ischaemic (coronary) heart disease, myocardial ischaemia (angina), myocardial infarction, aneurismal disease, atheromatous peripheral vascular disease, aortoiliac disease, chronic and critical lower limb ischaemia, visceral ischaemia, renal artery disease, cerebrovascular disease, stroke, atherosclerotic retinopathy, thrombosis and aberrant blood clotting, and hypertension. Such conditions may be medical or veterinary conditions.

A suitable test compound may be a small chemical entity, peptide, antibody molecule or other molecule whose effect on abzyme activity is to be determined. Suitable test compounds may be selected from compound collections and designed compounds, for example using combinatorial chemistry as described below. Particular suitable test compounds include metal chelators, for example copper or calcium chelators, antioxidants, in particular low pH anti-oxidants (i.e. compounds which inhibit oxidation reactions at pH 5.5), hydroxyl radical scavengers, 'electron trappers' such as crown ethers or steroids, 'electron cushions' such as polyvinyl-based polymers and 'electron sinks', such as ubiquinones and Q₈.

Suitable test compounds may include analogues, salts and derivatives of sodium azide, catechins, including catechin gallate, DMSO, azithromycin, haemoglobin and telithromycin, which have been identified as abzyme inhibitors using the present methods and fractions, extracts and derivatives of bacterial cells, in particular, cultures of probiotic bacteria such as lactobacilli.

Combinatorial library technology (Schultz, JS (1996) Biotechnol. Prog. 12:729-743) provides an efficient way of testing a potentially vast number of different substances for ability to modulate the activity of abzymes. Prior to or as well as being screened as described above, test compounds may be screened for ability to bind with the abzyme. This may be used as a coarse screen prior to testing a compound for actual ability to modulate abzyme activity.

The amount of test compound which may be added to an assay of the invention will normally be determined by trial and error depending upon the type of compound used. Typically, from about 0.01 to 100 nM concentrations of putative inhibitor compound may be used, for example from 0.1 to 10 nM. Compounds which may be used may be natural or synthetic chemical compounds used in drug screening programmes. Extracts of plants which contain several characterised or uncharacterised components may be used or extracts, fractions or components of probiotic bacteria, such as lactobacilli.

Other candidate inhibitor compounds may be based on modelling the 3-dimensional structure of the abzyme and/or the lipid antigen to which it binds and using rational drug design to provide potential inhibitor compounds with particular molecular shape, size and charge characteristics.

A screening method described herein may further comprise determining the lipid oxidation activity of an abzyme in the presence of the test compound.

Lipid oxidation activity, including lipid peroxidation activity, may be determined by determining the oxidation of host lipid (i.e. lipid from the sample), lipid from a foreign antigen such as a Chlamydia cell, or lipid from another source, which may for example be added as part of an assay method. The accumulation of oxidation products or by-products, such as co-oxidised coupled reporter molecules, may be measured or the disappearance or consumption of substrates such as non-modified lipids or co-substrates such as oxygen. Many methods for determining lipid peroxidation are known in the art and are suitable for use in accordance with the present invention. Suitable methods are, for example, described in CRC Handbook of Methods for Oxygen Radical Research, CRC Press, Boca Raton, Florida (1985), Oxygen Radicals in Biological Systems. Methods in Enzymology, v. 186, Academic Press, London (1990); Oxygen Radicals in Biological Systems. Methods in Enzymology, v. 234, Academic Press, San Diego, New York, Boston, London (1994); and, Free Radicals. A practical approach. IRL Press, Oxford, New York, Tokyo (1996) In preferred embodiments, oxidation is determined by determining the production (i.e. the presence or amount) of lipid oxidation products, include aldehydes such as malondialdehyde (MDA), (lipid) peroxides, diene conjugates or hydrocarbon gases. Lipid oxidation products may be determined by any suitable method. For example, lipid peroxidation products may be determined using HPLC (Brown, R.K., and Kelly, F.J In: Free Radicals. A practical approach. IRL Press, Oxford, New York, Tokyo (1996), 119-131), UV spectroscopy (Kinter, M. Quantitative analysis of 4-hydroxy-2-nonenal. Ibid., 133-145), or gas chromatography-mass spectrometry (Morrow, J.D., and Roberts, L.J. F₂-Isoprostanes: prostaglandin-like products of lipid peroxidation. Ibid. 147-157) . The production of malondialdehyde (MDA), for example, may be determined, following reaction with 2-thiobarbituric acid (conveniently at 1mM), by measuring absorbance at an appropriate wavelength, for example 525 nm.

An agent identified using one or more primary screens (e.g. in a cell-free system) as having ability to inhibit abzyme activity may be assessed further using one or more secondary screens. A secondary screen may involve testing for abzyme activity in the vascular system or for a biological function of an abzyme, for example, in an animal model. Suitable biological functions which may be assessed in a secondary screen include reduction in size or number of atherosclerotic lesions, or a reduction in other symptoms or effects of an atherosclerotic disorder, such as blood pressure.

Methods of the present invention may include identifying a test compound as an agent that inhibits anti-Chlamydia abzyme activity.

Examples of compounds identified as abzyme inhibitors using the present methods include ascorbic acid, acetyl salicylic acid, sodium azide, (+) catechin gallate, DMS, haemoglobin, telithromycin-ketek^{™}, lactobacillus cells and other agents as set out in Table 3.

The identified compound may be isolated or purified and/or synthesised or manufactured.

Optionally, a compound identified as anti-Chlamydia abzyme inhibitor as described herein may be modified to optimise activity or provide other beneficial characteristics such as increased half-life or reduced side effects upon administration to an individual. Techniques and strategies for the modification of lead compounds are well known in the art.

An abzyme inhibitor identified described herein may be formulated into a composition, such as a medicament, pharmaceutical composition or drug, with a pharmaceutically acceptable excipient as described below. Such a composition may be administered to an individual.

A compound may be identified using an assay method described above as abzyme inhibitor, a pharmaceutical or veterinary composition, medicament, drug or other composition may comprise such a compound, and a method may comprise administration of such a composition to a patient, e.g. for treatment (which may include preventative treatment) of atherosclerotic conditions. A compound may be used in manufacture of a composition for administration, e.g. for treatment of an atherosclerotic condition. A method of making a pharmaceutical or veterinary composition may comprise admixing such a compound with a pharmaceutically acceptable excipient, vehicle or carrier, and optionally other ingredients.

Whether it is a polypeptide, peptide, nucleic acid molecule, small molecule or other pharmaceutically useful compound that may be given to an individual, administration may be preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors.

A composition may be administered alone or in combination with other treatments, either simultaneously or sequentially dependent upon the condition to be treated.

Pharmaceutical compositions described may include, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous or intravenous.

Pharmaceutical compositions for oral administration may be in tablet, capsule, powder or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient may be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, or Lactated Ringer's Injection. Preservatives, stabilisers, buffers, antioxidants and/or other additives may be included, as required.

Those of skill in the art may vary the precise format of assay methods of the invention using routine skill and knowledge.

Various further aspects and embodiments of the present invention will be apparent to those skilled in the art in view of the present disclosure.

Certain aspects and embodiments of the invention will now be illustrated by way of example and with reference to the figures and tables described below.

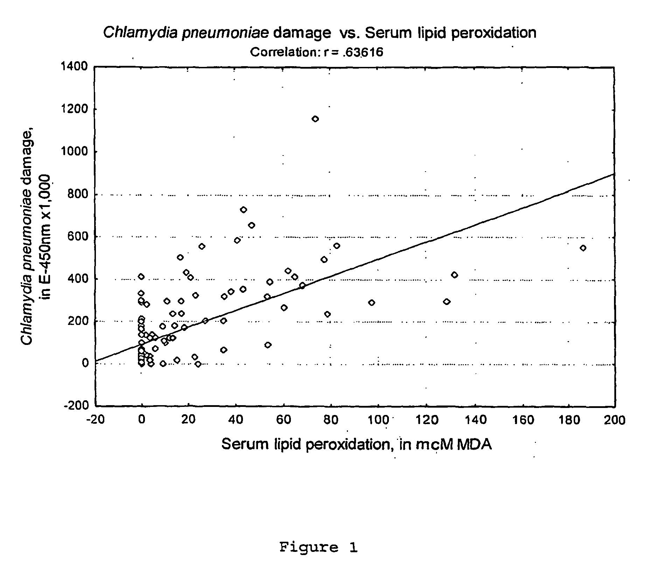

Figure 1 shows a comparison of lipid oxidation and Chlamydia antigen damage assays to measure the activity of anti-*Chlamydia* abzymes,
Table 1 shows a comparison of the measurement of the activity of anti-*Chlamydia* abzymes either via their ability to oxidise serum lipids or via their ability to damage *Chlamydia* antigen, ELISA assay,
Table 2 shows a comparison of the measurement of the activity of anti-*Chlamydia* abzymes either via their ability to oxidise serum lipids or via their ability to damage *Chlamydia* antigen, MIF assay.
Table 3 shows the effect of different factors on the ability of the abzymes to cause lipid peroxidation and damage the *Chlamydia pneumoniae* antigen(s).

### Examples

### Materials and Methods

### Preparation of Samples

Antibodies were extracted from advanced atherosclerotic lesions of human aorta retrieved from two male patients of 53 and 64 years old, during bypass surgery of an abdominal aortal stenosis at the Centre of Cardio-Vascular Surgery of the Medical University of Rostov-na-Donu, Russian Federation. After recovery these samples were immediately put in 30% w/v solution of NaCl and stored at 0-4°C for 1 month prior to examination. In the control experiments it was shown that during this period, the activities of such enzymes as trypsin, catalase, superoxide dismutase, glutathione peroxidase, creatine kinase and lactate dehydrogenase, together with a level of immunoglobulin (IgG) fragmentation and the degree of lipid peroxidation did not significantly change.

The pieces of aorta (approximately 200-400 mg wet weight) were cut into pieces of approximately 10mg each, placed in 5.0ml of PBS with 1% non-ionic detergent Igepal CA-630 and homogenised by a mechanical homogeniser (Ultra-Turrax) at full-power with a 15mm probe three times for 3 seconds each with 20 second cooling intervals. After homogenisation the insoluble components were separated by centrifugation at 5000g for 10 minutes and supernatants were used for analysis

The supernatant was treated with protein A attached to crosslinked 4% beaded agarose at 37°C for 30 minutes. The immunoglobulin fraction attached to the beads was then spun down at 5000g for 10 minutes and the supernatant decanted. In order to remove any lipoproteins attached to the sedimented immunoglobulins, the samples were re-suspended with 10% of Igepal CA-630. They were then centrifuged at 5000g for 10 minutes and the supernatant was decanted.

To remove the detergent three subsequent washings were performed in the excess of the phosphate buffer with centrifugation under the same regime. The removal of lipoprotein from the immunoglobulin fraction was confirmed by the absence of cholesterol in this fraction.

### Inactivation of abzymes

For physical inactivation, the same abzyme sample was split into two aliquots. One aliquot was heated in a water bath for 30 minutes at 56°C. The other was untreated. Following treatment of the first aliquot, both samples were tested in the same fashion.

For chemical inactivation, a diluent solution was divided into two portions. In one portion, an abzyme inhibitor was added. The following abzyme inhibitors were used DMSO, 0.1-10%; sodium azide, 10⁻⁵-10⁻³M; catechins, 10⁻⁶-10⁻³M; ketek, 10⁻⁶-10⁻³M; lactobacilli culture, 1µM-1mM; ascorbic acid, 10⁻⁴-10⁻³M; acetyl salicylic acid, 10⁻⁴-10⁻³M.

The serum sample was split in two aliquots and one aliquot was diluted by solution containing the abzyme inhibitor, the second aliquot was diluted by the control solution. The aliquots were then tested in the same fashion.

### ELISA based Abzyme Assay

ELISA assays were performed using Medac materials and reagents, which were used in accordance with the manufacturers instructions.

Briefly, serum samples from patients were treated as described above. 50 µl of sample diluent was pipetted into microtitre well A1 as blank, and 50 µl of the negative control, Positive Control and the diluted patients' samples were pipetted into other microtitre wells. The microplate wells were incubated for 60 min (± 5 min) at 37oC (± 1°C) in a humid chamber and then washed three times with 200µl wash buffer per well. 50 µl of Conjugate was then added to each well and the microplate wells incubated again for 60 min (± 5 min) at 37oC (± 1°C) in a humid chamber and then washed. 50 µl of TMB-Substrate, was added to each well and the microplate wells incubated for 30 min (± 2 min) at 37oC (± 1°C) in a humid chamber. The reaction was stopped by adding 100 µl of Stop Solution, to each well.

Photometric reading was performed at 450 nm (ref. 620 - 650 nm) within 15 min after adding the Stop Solution.

To calculate the results, the OD value of the blank (well Al) was subtracted from all other OD values. Preferably, the OD value of the blank was < 0.150, the mean OD value of the Negative Control was < 0.100 and the OD value of the Positive Control was > 0.800. Cut-off = mean OD value of the Negative Control + 0.380. Grey zone = Cut-off ± 10%

### Microimmunofluorescence (MIF) on lysed Chlamydia

Slides with antigens of *Chlamydia trachomatis, C. psittaci,* and *C. pneumoniae* were prepared by applying purified elementary bodies of these bacteria. Sera were diluted to a titer of 1:1024 in phosphate-buffered saline (PBS) and incubated for 30 min at 37°C. After washing in PBS, antihuman IgG, IgA, IgM conjugates were added to the samples. After 30 mins of incubation at 37°C and being washed in PBS, the slide was covered with a cover slip with mounting medium.

A fluorescent microscope was used for the reading of the slides. A positive reaction is represented by a "starry sky" appearance: fluorescent green spots on a slightly red background.

Two independent experts evaluated all samples.

### Analysis of ELISA and MIF Results

If there was a difference less than 10% (or no increase in titers in MIF) of the signal between treated and non-treated samples the conclusion was that abzyme activity was negative.

If there was a difference between 10 and 15% (or an increase in one titer in MIF) of the signal between treated and non-treated samples the conclusion was that the activity of the abzymes was unclear, or in the "grey" zone.

If there were a difference of more than 15% (or an increase in two titers or more in MIF) of the signal between treated and non-treated samples the conclusion was that the abzyme activity was positive.

### Electron microscopy on lysed Chlamydia

Bacteria cells were fixed for 1 hour in 2,5 % solution of glutaraldehyde, made in 0,2 M cacodylic buffer pH 7,2, after that in chrome-osmium solution for another hour.

After that samples were dehydrated in a gradient increase of ethanol and absolute acetone and imbedded in Eponate 12T14 - Araldite 502. Ultra-thin slides were made by using Ultracut Reichert - Jung, stained by 1 % water solution of uranyl acetate and lead citrate.

Slides were examined and photographed using an electron microscope JEM 100C × (with magnification of) × 5300-53000 times.

### SDS-PAGE

Polyacrylamide gel electrophoresis was performed using various commercially available systems, in accordance with the manufacturer's instructions. For example, the method described in DPO 033/02; Issue 1.0 "Protein electrophoresis using NOVEX^{™} system (SDS-PAGE)" was used withthe following reagents: NuPAGE^{™} Bis-Tris 4-12% precast gels (Invitrogen NP0321 batch #2063076) (15 well); NuPAGE^{™} Bis-Tris 4-12% precast gels (Invitrogen NP0321 batch #2072272) (10 well); NuPAGE^{™}LDS sample buffer 4x (Invitrogen NP0007 batch #300277); NuPAGE^{™} Sample reducing agent x10 (Invitrogen NP0004 batch #300505) NuPAGE^{™} MOPS SDS running buffer x20 (Invitrogen NP0001 batch #300704); SeeBlue^{™} pre stained markers (Invitrogen LC5625 batch #see11214).

### Determination of peroxidation of lipids

Lipid peroxidation was assessed as a level of MDA concentration, which was measured by spectrophotometric methods [Draper, H.H. et al Free Radic. Biol. Med. (1993) 15, 353]. Briefly, the level of abzymes in a sample was determined as follows: Samples of sera were diluted 1:1 by 0.05M acetate buffer pH 4.0 to make the final pH of these samples between 5.6-5.8. 990µl of the diluted serum was mixed with 10µl of the commercial live ovine *Chlamydia* vaccine (Intervet). Samples were incubated overnight (12-16 hours) at 37°C. 250µl of 40% trichloroacetic acid and 250µl of 1mM 2-thiobarbituric acid was added to each sample. All samples were placed in a water bath and boiled for 30 minutes. Samples were cooled down and centrifuged at 3,000g for 10 minutes. The supernatants were collected and their absorption measured at λ 525nm to determine the concentration of malondialdehydes (MDA), which are products of lipid peroxidation.

### Results

### Effect of Abzymes on Chlamydia

An abzyme-enriched fraction of atheroma IgG was prepared as described above. The effect of the abzyme-enriched fraction of atheroma IgG on *Chlamydia pneumoniae* elementary and recticulocyte bodies was determined by MIF and electron microscopy.

The abzyme-enriched fraction was observed to lyse *Chlamydia pneumoniae* cells in a microimmunofluorescence assay (MIF). Electron microscopy showed that the abzyme-enriched fraction lysed both *Chlamydia pneumoniae* elementary and reticular bodies.

### Analysis of Abzyme-enriched fraction of atheroma IgG

The abzyme-enriched fraction of atheroma IgG was analysed by SDS-PAGE along with an IgG fraction purified from serum.

Electrophoresis analysis showed that the abzyme-enriched fraction contained only IgG and no any other detectable proteins.

### Abzyme assays

ELISA and MIF assays for Chlamydia antigen damage as described above were used to measure the activity of anti-*Chlamydia* abzymes in patient serum samples, in comparison with lipid oxidation assays. The results are set out in tables 1 and 2, respectively and summarised in Figure 1.

The results of the ELISA (in E₄₅₀ₙₘ×1,000) and MIF (Ab titer) assays were observed to correlate with the results of lipid peroxidation assays.

### Abzyme Inhibition

A range of treatments were tested for ability to inactivate abzymes by ELISA as described above and the lipid oxidation assay for ability to inhibit abzyme activity. The results are set out in table 3.

Ascorbic acid, acetyl salicylic acid, sodium azide, EDTA, EGTA, catechin gallate, DMSO, haemoglobin, telithromycin ketek, and lactobacilli were all observed to inhibit abzyme activity.

Compounds which inactivate abzymes may be useful in methods of for determining abzyme levels or may be useful in therapy, for example the treatment of atherosclerotic or cardiovascular conditions.

**Table 1**

| Abzyme-negative sera (n = 33), measurement of the activity based on: | | Abzyme positive sera (n = 33) measurement of the activity based on: | |
|---|---|---|---|
| serum peroxidation, in µM MDA | *Chlamydia pneumoniae* antigen damage, in ELISA E₄₅₀ₙₘ×1,000 | serum peroxidation, in µM MDA | *Chlamydia pneumoniae* antigen damage, in ELISA E₄₅₀ₙₘ×1,000 |
| 0 | 139 | 186 | 550 |
| 6 | 271 | 128 | 296 |
| 4 | 136 | 37 | 283 |
| 9 | 307 | 35 | 207 |
| 3 | 15 | 131 | 424 |
| 0 | 0 | 54 | 390 |
| 0 | 31 | 82 | 564 |
| 0 | 0 | 77 | 494 |
| 0 | 8 | 47 | 660 |
| 0 | 16 | 35 | 322 |
| 0 | 63 | 27.5 | 206 |
| 0 | 43 | 38 | 345 |
| 0 | 0 | 43.5 | 732 |
| 3 | 124 | 41 | 584 |
| 0 | 61 | 65 | 413 |
| 0 | 100 | 60 | 267 |
| 0 | 166 | 26 | 555 |
| 9 | 278 | 19 | 432 |
| 0 | 8 | 78.5 | 241 |
| 0 | 28 | 62 | 443 |
| 0 | 0 | 21 | 409 |
| 0 | 4 | 12 | 124 |
| 0 | 11 | 23 | 324 |
| 0 | 11 | 18 | 172 |
| 0 | 14 | 17 | 298 |
| 0 | 16 | 97.5 | 293 |
| 0 | 26 | 13 | 237 |
| 0 | 204 | 43 | 356 |
| 0 | 6 | 53 | 320 |
| 0 | 0 | 74 | 1158 |
| 10 | 99 | 68 | 376 |
| 0 | 0 | 24 | 376 |
| 0 | 0 | 17 | 238 |
| 1. 3 ± 0.025 | 58 ± 13.7 | 52.8 ± 6.17 | 388 ± 31.4 |
| | | p < 0.001 | p < 0.001 |

**Table 2**

| Patients | Abzyme activity in terms of the loss of the antibody ability to damage *Chlamydia pneumoniae* antigen | |
|---|---|---|
| | MIF titers before abzyme inactivation | MIF titers after abzyme inactivation |
| Abzyme (+) sera, tested by lipid oxidation MDA assay | | |
| P577 | 0 | 1/128 |
| OAG | 0 | 1/64 |
| YIO | 0 | 1/64 |
| IVM | 0 | 1/64 |
| IMK | 0-1/16 | 1/64 |
| P580 | 0 | 1/64 |
| P573 | 0 | 1/64 |
| P571 | 0 | 1/32 |
| AFP | 0 | 1/16-1/32 |
| VAM | 0 | 1/16 |
| P572 | 0 | 0 |

| Abzyme (-) sera, tested by lipid oxidation MDA assay | | |
|---|---|---|
| P585 | 0 | 0 |
| AIS | 0 | 0 |
| GPM | 0 | 0 |
| P567 | 0 | 0 |
| INK | 0 | 0-1/16 |
| SII | 0 | 0-1/16 |
| NEC | 0 | 0 |
| JON | 0 | 0-1/16 |
| KAT | 0 | 0 |
| JIM | 0 | 0 |

**Table 3**

| Factors affecting abzyme activity | Inhibition of abzymes ability to cause: | |
|---|---|---|
| | Serum lipid peroxidation, in MDA assay | Damage of *Chlamydia pneumoniae* antigen, in ELISA |
| Physical procedures | | |
| Repetitive freezing thawing | Positive | Positive |
| Heating at 56°C for 30 min | Positive | Positive |

| Drugs, reagents or food products | | |
|---|---|---|
| 1. Acetyl salicylic acid | Positive | Positive |
| 2. Ascorbic acid | Positive | Positive |
| 3. EDTA | Positive | Positive |
| 4. EGTA | n/a | Positive |
| 5. Sodium cyanide | Negative | Negative |
| 6. Sodium azide | Positive | Positive |
| 7. (+) Catechin gallate | Positive | Positive |
| 8. β-Carotene | Negative | Negative |
| 9. (+) α-Tocopherol | Negative | Negative |
| 10. (+) γ-Tocopherol | Negative | Negative |
| 11. Benzoic acid | Negative | Negative |
| 12. DMSO | Positive | Positive |
| 13. D-Mannitol | Negative | Negative |
| 14. PMS | Negative | Negative |
| 15. Haemoglobin | Positive | Positive |
| 16. Telithromycin, Ketek^{TM} | Positive | Positive |
| 17. Tetracycline | Negative | Negative |
| 18. Lactobacilli culture | Positive | Positive |
| 19. Lycopene | Negative | Negative |

## Claims

1. A method of measuring lipid-oxidising anti-Chlamydia abzyme levels in a sample comprising;
abolishing anti-Chlamydia abzyme-mediated lipid oxidation in said sample without affecting the binding of abzymes or anti-Chlamydia antibodies to Chlamydia antigens, and
determining the binding of antibodies in the sample to a Chlamydia antigen,
wherein an increase in binding in the treated sample relative to controls is indicative of the presence or level of abzymes in the sample.

2. A method according to claim 1 wherein the sample is a blood, serum or plasma sample obtained from an individual.

3. A method according to claim 2 wherein the presence of abzymes in the sample is indicative of an atherosclerotic condition.

4. A method according to any one of the preceding claims wherein the sample is physically treated to abrogate abzyme-mediated lipid oxidation.

5. A method according to claim 4 wherein the sample is heated.

6. A method according to claim 5 wherein the sample is heated to at least 37°C for at least 8 hours.

7. A method according to claim 6 wherein the sample is heated to at least 56°C for at least 30 minutes.

8. A method according to claim 4 wherein the sample is exposed to two or more freeze thaw cycles.

9. A method according to claim 4 wherein the sample is maintained at 0°C to 4°C for at least 4 days.

10. A method according to any one of claims 1 to 3 wherein the sample is chemically treated to abrogate abzyme-mediated lipid oxidation.

11. A method according to claim 10 wherein the sample is treated with one or more inactivating agents.

12. A method according to claim 11 wherein the inactivating agent is a hydroxyl radical scavenger or low pH anti-oxidant.

13. A method according to claim 11 wherein the inactivating agent is a hydroxyl radical scavenger.

14. A method according to any one of claims 11 to 13 wherein the inactivating agent is selected from the group consisting of acetyl salicylic acid, ascorbic acid, ethylenediaminetetraacetic acid (EDTA), ethyleneglycoltetraacetic acid (EGTA), (+) catechin gallate, sodium azide, dimethyl sulfoxide (DMSO), haemoglobin and telithromycin.

15. A method according to claim 11 wherein the inactivating agent is a bacterial cell.

16. A method according to claim 15 wherein the inactivating agent is a lactobacillus cell.

17. A method according to any one of the preceding claims wherein the amount of abzyme mediated lipid oxidation in the sample is determined after said treatment.

18. A method according to any one of the preceding claims wherein the Chlamydia antigen is on the surface of a Chlamydia cell.

19. A method according to any one of the preceding claims wherein the binding of the antibody to the Chlamydia antigen is determined using a second antibody.

20. A method according to claim 19 wherein the second antibody binds to Immunoglobulin G (IgG).

21. A method according to claim 19 or claim 20 wherein one of the second antibody and the Chlamydia antigen or cell is labelled.

22. A method according to claim 21 wherein the other of the second antibody and the Chlamydia antigen or cell is immobilised.

23. A method of screening for an inhibitor of anti-Chlamydia abzyme-mediated lipid oxidation comprising;
determining the binding of antibodies in a sample to a Chlamydia antigen,
wherein the sample comprises lipid oxidising anti-Chlamydia abzymes,
treating the sample with a test compound,
wherein the test compound does not affect the binding of abzymes or antibodies to a Chlamydia antigen, and
determining the binding of antibodies in the treated sample to a Chlamydia antigen,
an increase in binding in the sample treated with the test compound relative to untreated sample being indicative that the compound is an inhibitor of abzyme-mediated lipid oxidation.

24. A method according to claim 23 wherein the sample was obtained from an individual having an atherosclerotic disorder.

25. A method according to claim 24 wherein the sample is a serum or atheroma sample.

26. A method according to any one of claims 23 to 25 wherein the sample is an IgG enriched sample.

27. A method according to any one of claims 23 to 26 wherein the test compound is a low pH antioxidant.

28. A method according to any one of claims 23 to 26 wherein the test compound is a hydroxyl radical scavenger.

29. A method according to any one of claims 23 to 28 comprising determining the lipid oxidation activity of an abzyme in the presence of the test compound.

## Patentansprüche

1. Verfahren zur Messung der Spiegel von Lipid oxidierendem Anti-Chlamydia-Abzym in einer Probe, umfassend:
das Aufheben von durch Anti-Chlamydia-Abzym vermittelter Lipidoxidation in der Probe, ohne dabei die Bindung von Abzymen oder Anti-Chlamydia-Antikörpern an Chlamydia-Antigene zu beeinträchtigen, und
das Bestimmen der Bindung von Antikörpern in der Probe an ein Chlamydia-Antigen,
worin eine Erhöhung der Bindung in der behandelten Probe gegenüber Kontrollen ein Indikator für das Vorhandensein oder einen Spiegel von Abzymen in der Probe ist.

2. Verfahren nach Anspruch 1, worin die Probe eine einem Individuum entnommene Blut-, Serum- oder Plasmaprobe ist.

3. Verfahren nach Anspruch 2, worin das Vorhandensein von Abzymen in der Probe auf ein atherosklerotisches Leiden hindeutet.

4. Verfahren nach einem der vorangegangenen Ansprüche, worin die Probe physikalisch behandelt wird, um abzymvermittelte Lipidoxidation aufzuheben.

5. Verfahren nach Anspruch 4, worin die Probe erhitzt wird.

6. Verfahren nach Anspruch 5, worin die Probe für zumindest 8 h auf zumindest 37 °C erhitzt wird.

7. Verfahren nach Anspruch 6, worin die Probe für zumindest 30 min auf zumindest 56 °C erhitzt wird.

8. Verfahren nach Anspruch 4, worin die Probe zwei oder mehr Gefrier-Auftau-Zyklen unterzogen wird.

9. Verfahren nach Anspruch 4, worin die Probe zumindest 4 Tage lang bei 0 °C bis 4 °C aufbewahrt wird.

10. Verfahren nach einem der Ansprüche 1 bis 3, worin die Probe chemisch behandelt wird, um abzymvermittelte Lipidoxidation aufzuheben.

11. Verfahren nach Anspruch 10, worin die Probe mit einem oder mehreren Deaktivierungsmitteln behandelt wird.

12. Verfahren nach Anspruch 11, worin das Deaktivierungsmittel ein Hydroxylradikalfänger oder ein Antioxidationsmittel mit niedrigem pH-Wert ist.

13. Verfahren nach Anspruch 11, worin das Deaktivierungsmittel ein Hydroxylradikalfänger ist.

14. Verfahren nach einem der Ansprüche 11 bis 13, worin das Deaktivierungsmittel aus der aus Acetylsalicylsäure, Ascorbinsäure, Ethylendiamintetraessigsäure (EDTA), Ethylenglykoltetraessigsäure (EGTA), (+)-Catechingallat, Natriumazid, Dimethylsulfoxid (DMSO), Hämoglobin und Telithromycin bestehenden Gruppe ausgewählt ist.

15. Verfahren nach Anspruch 11, worin das Deaktivierungsmittel eine BakterienZelle ist.

16. Verfahren nach Anspruch 15, worin das Deaktivierungsmittel eine Lactobacillus-Zelle ist.

17. Verfahren nach einem der vorangegangenen Ansprüche, worin die Menge an abzymvermittelter Lipidoxidation in der Probe nach der Behandlung bestimmt wird.

18. Verfahren nach einem der vorangegangenen Ansprüche, worin das Chlamydia-Antigen sich an der Oberfläche einer Chlamydia-Zelle befindet.

19. Verfahren nach einem der vorangegangenen Ansprüche, worin die Bindung des Antikörpers an das Chlamydia-Antigen unter Verwendung eines zweiten Antikörpers bestimmt wird.

20. Verfahren nach Anspruch 19, worin der zweite Antikörper an Immunglobulin G (IgG) bindet.

21. Verfahren nach Anspruch 19 oder Anspruch 20, worin von dem zweiten Antikörper und dem Chlamydia-Antigen bzw. der Chlamydia-Zelle eines markiert ist.

22. Verfahren nach Anspruch 21, worin von dem zweiten Antikörper und dem Chlamydia-Antigen bzw. der Chlamydia-Zelle das jeweils andere immobilisiert ist.

23. Verfahren zum Screenen nach einem Inhibitor von durch ein Anti-Chlamydia-Abzym vermittelter Lipidoxidation, umfassend:
das Bestimmen der Bindung von Antikörpern in einer Probe an ein Chlamydia-Antigen,
worin die Probe Lipid oxidierende Anti-Chlamydia-Abzyme umfasst,
das Behandeln der Probe mit einer Testverbindung,
worin die Testverbindung die Bindung von Abzymen oder Antikörpern an ein Chlamydia-Antigen nicht beeinträchtigt, und
das Bestimmen der Bindung von Antikörpern in der behandelten Probe an ein Chlamydia-Antigen,
wobei eine Zunahme der Bindung in der mit der Testverbindung behandelten Probe gegenüber unbehandelter Probe ein Indikator dafür ist, dass es sich bei der Verbindung um einen Inhibitor von abzymvermittelter Lipidoxidation handelt.

24. Verfahren nach Anspruch 23, worin die Probe von einem Individuum mit einer atherosklerotischen Erkrankung stammt.

25. Verfahren nach Anspruch 24, worin die Probe eine Serum- oder Atheromprobe ist.

26. Verfahren nach einem der Ansprüche 23 bis 25, worin die Probe eine an IgG angereicherte Probe ist.

27. Verfahren nach einem der Ansprüche 23 bis 26, worin die Testverbindung eine Antioxidationsmittel mit niedrigem pH-Wert ist.

28. Verfahren nach einem der Ansprüche 23 bis 26, worin die Testverbindung ein Hydroxylradikalfänger ist.

29. Verfahren nach einem der Ansprüche 23 bis 28, umfassend das Bestimmen der Lipidoxidationsaktivität eines Abzyms in Gegenwart der Testverbindung.

## Revendications

1. Procédé pour mesurer des niveaux d'abzyme anti-Chlamydia d'oxydation des lipides dans un échantillon, comprenant:
abolir l'oxydation des lipides anti-Chlamydia régulée par les abzymes dans ledit échantillon sans affecter la liaison des abzymes ou anticorps anti-Chlamydia à des antigènes de Chlamydia, et
déterminer la liaison des anticorps dans l'échantillon à un antigène de Chlamydia,
où une augmentation dans la liaison dans l'échantillon traité relativement à des contrôles est indicative de la présence ou du niveau d'abzymes dans l'échantillon.

2. Procédé selon la revendication 1, dans lequel l'échantillon est un échantillon de sang, de sérum ou de plasma obtenu d'un individu.

3. Procédé selon la revendication 2, dans lequel la présence d'abzymes dans l'échantillon est indicative d'un état athérosclérotique.

4. Procédé selon l'une quelconque des revendications précédentes, dans lequel l'échantillon est traité physiquement pour abroger l'oxydation des lipides régulée par les abzymes.

5. Procédé selon la revendication 4, dans lequel l'échantillon est chauffé.

6. Procédé selon la revendication 5, dans lequel l'échantillon est chauffé à au moins 37°C pendant au moins 8 heures.

7. Procédé selon la revendication 6, dans lequel l'échantillon est chauffé à au moins 56°C pendant au moins 30 minutes.

8. Procédé selon la revendication 4, dans lequel l'échantillon est exposé à deux ou plusieurs cycles gel-dégel.

9. Procédé selon la revendication 4, dans lequel l'échantillon est maintenu à 0°C jusqu'à 4°C pendant au moins 4 jours.

10. Procédé selon l'une quelconque des revendications 1 à 3, dans lequel l'échantillon est traité chimiquement pour abroger l'oxydation des lipides régulée par les abzymes.

11. Procédé selon la revendication 10, dans lequel l'échantillon est traité avec un ou plusieurs agents d'inactivation.

12. Procédé selon la revendication 11, dans lequel l'agent d'inactivation est un désactiveur à radical hydroxyle ou un antioxydant d'un pH faible.

13. Procédé selon la revendication 11, dans lequel l'agent d'inactivation est un désactiveur à radical hydroxyle.

14. Procédé selon l'une quelconque des revendications 11 à 13, dans lequel l'agent d'inactivation est sélectionné dans le groupe consistant en acide acétyl salicylique, acide ascorbique, acide éthylènediaminetétraacétique (EDTA), acide éthylèneglycotétraacétique (EGTA), (+) catéchin gallate, sodium azide, diméthyl sulfoxyde (DMSO), hémoglobine et télithromycine.

15. Procédé selon la revendication 11, dans lequel l'agent d'inactivation est une cellule bactérienne.

16. Procédé selon la revendication 15, dans lequel l'agent d'inactivation est une cellule *lactobacillus.*

17. Procédé selon l'une quelconque des revendications précédentes, dans lequel la quantité d'oxydation des lipides régulée par les abzymes dans l'échantillon est déterminée après ledit traitement.

18. Procédé selon l'une quelconque des revendications précédentes, dans lequel l'antigène de Chlamydia se trouve sur la surface d'une cellule Chlamydia.

19. Procédé selon l'une quelconque des revendications précédentes, dans lequel la liaison de l'anticorps à l'antigène de Chlamydia est déterminée en utilisant un deuxième anticorps.

20. Procédé selon la revendication 19, dans lequel le deuxième anticorps se lie à l'immunoglobuline G (IgG).

21. Procédé selon la revendication 19 ou la revendication 20, dans lequel un parmi le deuxième anticorps et l'antigène de Chlamydia ou la cellule est marqué.

22. Procédé selon la revendication 21, dans lequel l'autre parmi le deuxième anticorps et l'antigène de Chlamydia ou la cellule est immobilisé.

23. Procédé de criblage d'un inhibiteur d'une oxydation des lipides anti-Chlamydia régulée par des abzymes comprenant:
déterminer la liaison des anticorps dans un échantillon à un antigène de Chlamydia,
où l'échantillon comprend des abzymes anti-Chlamydia d'oxydation des lipides,
traiter l'échantillon avec un composé test,
où le composé test n'agit pas sur la liaison des abzymes ou anticorps à un antigène de Chlamydia, et
déterminer la liaison des anticorps dans l'échantillon traité à un antigène de Chlamydia,
une augmentation dans la liaison dans l'échantillon traité avec le composé test relativement à l'échantillon non traité étant indicative de ce que le composé est un inhibiteur de l'oxydation des lipides régulée par les abzymes.

24. Procédé selon la revendication 23, dans lequel l'échantillon a été obtenu d'un individu présentant un trouble athérosclérotique.

25. Procédé selon la revendication 24, dans lequel l'échantillon est un sérum ou un échantillon d'athérome.

26. Procédé selon l'une quelconque des revendications 23 à 25, dans lequel l'échantillon est un échantillon enrichi en IgG.

27. Procédé selon l'une quelconque des revendications 23 à 26, dans lequel le composé test est un antioxydant d'un pH bas.

28. Procédé selon l'une quelconque des revendications 23 à 26, dans lequel le composé test est un désactiveur à radical hydroxyle.

29. Procédé selon l'une quelconque des revendications 23 à 28, comprenant la détermination de l'activité de l'oxydation des lipides d'un abzyme en présence du composé test.
